# EUROPEAN PATENT APPLICATION

(11) **EP 4 825 974 A1**
(43) Date of publication of application: **21.10.2026**
(21) Application number: 25171185.9
(22) Date of filing: 17.04.2025
(51) Int. Cl.: C12P 17/16, B01J 8/00, C12M 1/42, C12M 1/00, C12N 13/00

(54) **METHOD OF INCREASING BACTERIAL PIGMENT PRODUCTION USING PLASMA-GENERATED RONS**

(71) Applicant: Jozef Stefan Institute, 1000 Ljubljana (SI)
(72) Inventor: MODIC, Martina, 1000 Ljubljana (SI); NORTHAGE, Naomi, 1000 Ljubljana (SI); HOJNIK, Natasa, 1000 Ljubljana (SI); CVELBAR, Uros, 1000 Ljubljana (SI)
(74) Representative: Weickmann & Weickmann PartmbB

(57) **Abstract**

The present invention relates to use of cold plasma to increase production of at least one secondary metabolite in a bacterial culture, a method for producing at least one secondary metabolite in a bacterial culture and a system for increasing production of at least one secondary metabolite in a bacterial culture.

## Description

### FIELD OF INVENTION

The present invention relates to use of cold plasma to increase production of at least one secondary metabolite in a bacterial culture, a method for producing at least one secondary metabolite in a bacterial culture and a system for increasing production of at least one secondary metabolite in a bacterial culture. Thus, the present invention generally relates to the increased production of bacterial secondary metabolites. More specifically, the invention provides a method for increasing the production of bacterial secondary metabolites, such as pigments, through the use of plasma-generated reactive oxygen and nitrogen species (RONS). The method is applicable to various bacterial strains capable of producing secondary metabolites, such as pigments, including, but not limited to, violacein. The invention includes optimal growth and media conditions, along with subsequent recovery and extraction of the pigment from the culture medium. It is presented that plasma-generated RONS can increase the production of bacterial secondary metabolites, including violacein, and potentially stimulate the production of other bacterial secondary metabolites.

### BACKGROUND OF THE INVENTION

Bacterial secondary metabolites are non-essential compounds not needed for growth and proliferation, but that can facilitate the interaction of a microbe with its environment and provide a competitive evolutionary advantage. Secondary metabolites are substances like pigments, alkaloids, terpenes, phenolic, nitrogenous compounds, essential oils, resins and toxins. They are usually specific to a species, genre, or branch. The formation of secondary metabolites usually occurs during stationary phase of growth and is suppressed during log phase (exponential growth), and environmental factors such as pH, nutrient availability, temperature and light can affect their production. For example, presence of high glucose concentrations can result in increased growth and suppression of the production of secondary metabolites. Secondary metabolites have become a prevalent feature of modern biotechnology, with metabolites being used as insecticides, flavourings, colourants, essences, antioxidants, and cosmetics. A major benefit to the use of bacterial secondary metabolites is the fact that they are natural compounds and may not be harmful to humans or the environment unlike their chemical counterparts, making them an advantageous alternative.

Secondary metabolites have a lot of potential and are currently produced using fermentation, genetic engineering, and co-cultivation processes. During the fermentation process, the temperature, pH, oxygen and nutrient availability of the bacterial culture is optimised to maximise the production of secondary metabolites. Whereas, with genetic engineering the production of metabolites is enhanced through techniques such as mutagenesis, recombinant DNA technology, or genome editing. Multiple bacterial strains are used in co-cultivation to trigger natural microbial interactions, resulting in an exchange of signalling molecules and metabolites, and thus increasing yields of metabolites. However, all these methods present significant challenges for large-scale production due to their complexity, high cost, and the need for precise environmental control. Often these processes can result in low and inconsistent yields.

An example of a bacterial secondary metabolites are pigments. Pigments are compounds produced by various bacterial species that contribute to their characteristic colours and serve a range of ecological and functional roles. These pigments, which include violacein, carotenoids, anthocyanins, and melanin, play vital roles in microbial metabolism, protection against environmental stressors, and communication within microbial communities. For example, pigments can function as antioxidants, providing protection from oxidative damage, along with acting as signalling molecules to facilitate species interactions. The diverse applications of bacterial pigments have led to increased attention and demand in textiles, cosmetics, and pharmaceuticals, offering a natural and often safer alternative to synthetic dyes and colorants. The growing interest in sustainable practices has further increased importance of research into optimizing the production of these pigments.

One example for a bacterial pigment which has attracted interest is the deep purple pigment violacein, which is natively produced by a number of bacterial strains including *Chromobacterium violaceum, Janthinobacterium lividum* and *Duganella.* Violacein has been shown to have significant potential, including antibacterial, antiviral, anticancer, antiparasitic, and antioxidant properties, making it a valuable compound for various applications. Current methods to increase violacein yield rely on manipulating environmental conditions or through genetic modifications of the bacterium.

US 11566265 B2 describes a method for the microbial production of terpenoids, examples of bacterial secondary metabolites, through the engineering of the isoprenoid biosynthetic pathway.

WO2011110932A1 discloses a process for the production of violacein and its derivative (deoxyviolacein) from *Chromobacterium* sp. NIIST (MTCC 5522). The method comprises of the steps of maintaining and growing the bacterium in a specific medium under defined conditions of pH, temperature and agitation.

CN101386834A discloses a method and a recombinant bacterial strain for producing violacein. Gene clusters related to the production of violacein were introduced into *Escherichia coli* and *Pseudomonas putida.* The method for producing the violacein pigment comprises use of L-tryptophan as a substrate and utilization of the recombinant strain to produce the violacein.

Production methods and applications of violacein are reviewed in Ahmed et al. (Ahmed et al. 2021, Journal of Microbiology and Biotechnology, 31(11), 1465-1480).

Choi et al. (Choi et al. 2015, Scientific Reports, 5(1), 15598) describe isolation of a new strain of *Duganella violaceinigra* with high violacein production. Kanelli et al. (Kanelli et al. 2018, Frontiers in Microbiology, 9) describe optimized conditions for production of violacein from *Janthinobacterium lividum,* including a pH of 7.0, temperature of 25°C, glycerol as carbon source and 0.1-0.4 mg/ml ampicillin.

Methods to increase secondary metabolite or specifically bacterial pigment production as described here, while promising, have several disadvantages as optimisation of culture conditions and genetic modification are time consuming and involve limited control of metabolite production which can result in low and inconsistent yields. In addition, these approaches require complex processes that may limit scalability and cost-effectiveness.

Therefore, a more universal, reliable, rapid, and scalable method is needed to increase bacterial secondary metabolite production efficiently.

The present inventors have found that cold atmospheric pressure plasma, herein referred to as cold plasma, may be a novel tool to increase bacterial secondary metabolite production, such as production of violacein. Cold plasma generates an abundance of RONS, such as ozone (O₃), atomic oxygen (O), superoxide (O₂⁻), hydroxyl radicals (OH), nitric oxide (NO), and hydrogen peroxide (H₂O₂). Plasma has been shown to be a formidable antimicrobial agent. However, according to the present invention, subinhibitory plasma exposure may alter bacterial metabolism in a matter of seconds without the need for genetic modification.

Thus, the present invention relates to the use of plasma generated RONS for altering bacterial metabolism to increase bacterial secondary metabolite production for biotechnological, industrial and pharmaceutical applications. Violacein, a deep purple pigment with antimicrobial and anticancer properties, serves as a model compound for studying RONS-induced metabolite production. However, the principles underlying RONS-induced pigment production can be extended to a broader range of bacterial secondary metabolites, making this approach applicable to various industrially relevant strains.

Accordingly, the present invention provides a surprising solution to the problem of providing universal means to influence and increase bacterial secondary metabolite production. The method of the invention increases bacterial secondary metabolite production in a matter of seconds using plasma-generated RONS as a targeted, non-genetic method for enhancing bacterial secondary metabolite production in a rapid, scalable and cost-effective manner. Unlike conventional methods, this approach does not require complex nutrient manipulation, extended incubation times, or genetic engineering of bacterial strains. By fine tuning plasma treatment parameters, RONS exposure can be optimised to maximize metabolite yield while maintaining bacterial viability. This method represents a significant advancement in microbial biotechnology.

### OBJECTIVE OF THE INVENTION

The main objective of the present invention is to provide a method for increasing the production of bacterial secondary metabolites, such as pigments, through the application of plasma-generated RONS. Specifically, the invention aims is to enhance the production of bacterial secondary metabolites, including but not limited to the pigment violacein, by exposing pigment-producing bacterial strains to controlled plasma treatments under optimized growth and media conditions. A further objective is to establish an efficient method for the recovery and extraction of pigments from bacterial cultures following plasma treatment. Additionally, the present invention aims to optimize culture conditions and plasma parameters to maximize metabolite yield, ensure reproducibility, and enable large-scale production of bacterial secondary metabolites.

### SUMMARY OF THE INVENTION

The present inventors have developed a method of increasing bacterial secondary metabolite production using plasma-generated RONS.

The method is applicable to various bacterial strains capable of producing secondary metabolites, including but not limited to pigments.

The invention includes optimal growth and media conditions, along with subsequent recovery and extraction of the pigment from the culture medium.

This approach allows for significantly shorter treatment times, in a more sustainable and cost-effective manner, making it suitable for practical large-scale production.

It is already known that cold plasma has antimicrobial activity, but recent studies suggest that controlled, subinhibitory exposure to plasma-generated RONS can influence bacterial metabolism and potentially alter the production of secondary metabolites. RONS can act as signalling molecules that trigger oxidative stress responses, influencing gene expression and metabolic pathways. The present invention exploits these effects to increase bacterial secondary metabolite production, using the production of the pigment violacein as a model case. The cold plasma device used in the present invention provides a novel approach for increasing bacterial secondary metabolite production by using an indirect cold plasma device. This system, featuring a mesh-grounded stainless-steel electrode and a quartz dielectric barrier, generates RONS in ambient air to stimulate bacterial metabolic pathways without direct contact. This ensures that longer-lived RONS interact with bacterial cultures in a controlled manner, promoting rapid metabolite production without genetic engineering or complex environmental control.

The invention describes a method for increasing secondary metabolite production in bacteria through exposure to controlled plasma-generated RONS under optimized culture conditions.

Thus, in a first aspect, the invention is directed to a method for producing at least one secondary metabolite, comprising the steps of:
(i) providing a bacterial culture capable of producing the at least one secondary metabolite,
(ii) treating the bacterial culture of step (i) with cold plasma, and
(iii) cultivating the bacterial culture obtained from step (ii), and optionally
(iv) extracting the secondary metabolite from the bacterial culture obtained in step (iii).

The metabolite concentration may be assessed using standard analytical techniques, such as absorbance measurements.

The bacterial culture is preferably a liquid bacterial culture and preferably maintained in a culture medium and/or growth medium. In preferred embodiments, the growth medium is tryptic soy broth (TSB) and consists of 2.5g glucose monohydrate, 5g sodium chloride, 2.5g dipotassium hydrogen phosphate, 17g pancreatic digest of casein, 3g papaic digest of soyabean meal per 1 L of distilled water.

In preferred embodiments of the present invention, *Janthinobacterium lividum* DSM1522 is maintained in TSB and exposed to low, medium and high-power cold plasma subinhibitory and inhibitory treatments. The yield of the violacein pigment produced is 5.83 times higher when *Janthinobacterium lividum DSM1522* maintained in TSB is exposed to a high-power plasma treatment.

Preferably, pigment production in *Janthinobacterium lividum* DSM1522 following cold plasma exposure may be measured after 24, 48 or 72 hours. Increased pigment production is sustained over longer incubation times.

Further, growth of *Janthinobacterium lividum* DSM1522 in response to low and high-power cold plasma exposure may be monitored over 24 hours. It was found by the inventors that cold plasma exposure disrupted normal growth of *Janthinobacterium lividum* DSM1522 and resulted in a subinhibitory or inhibitory response. High power plasma has a greater effect of growth of *Janthinobacterium lividum* DSM1522 than low power.

The inventors further compared UV exposure to cold plasma exposure in its effects on growth of *Janthinobacterium lividum* DSM1522. UV does not result in significant disruption of growth of *Janthinobacterium lividum* DSM1522 as seen with cold plasma. UV alone does not affect growth in comparison with cold plasma.

### BRIEF DESCRIPTION OF THE DRAWINGS

Embodiments illustrating the invention will now be discussed with reference to the attached figures, which show:
**Figure 1** shows a schematic of a system according to the present invention, comprising a cold plasma device for generation of RONS and a bacterial culture in a vessel, wherein the plasma is applied to the bacterial culture to increase production of bacterial secondary metabolites.
**Figure 2** shows bacterial pigment production following exposure to subinhibitory and inhibitory cold plasma treatments under different power settings (low, medium and high) after 24 hours.
**Figure 3** shows bacterial pigment production following exposure to subinhibitory and inhibitory cold plasma treatments under different power settings (low, medium and high) after 48 hours.
**Figure 4** shows bacterial pigment production following exposure to subinhibitory and inhibitory cold plasma treatments under different power settings (low, medium and high) after 72 hours.
**Figure 5** shows growth curves of bacterial cultures exposed to RONS from low power plasma treatment, showing effects on metabolism.
**Figure 6** shows growth curves of bacterial cultures exposed to RONS from high power plasma treatment, showing effects on metabolism.
**Figure 7** shows growth curves of bacterial cultures exposed to UV treatment, highlighting the effects of plasma-generated RONS compared to UV.

### DETAILED DESCRIPTION OF THE INVENTION

Aspects and embodiments of the present invention are now discussed with reference to the accompanying figures. Additional aspects and embodiments will be evident to those skilled in the art.

As used herein, a bacterial culture comprises a medium comprising a plurality of bacteria, preferably a liquid medium. A pure culture comprises a single bacterial strain, whereas a co-culture comprises two or more different strains, wherein the strains are preferably chosen to interact and promote secondary metabolite production. Bacterial strains mentioned herein are preferably aerobic bacteria, particularly obligate aerobes, facultative anaerobes or aerotolerant anaerobes.

As used herein, a native (bacterial) strain is a strain that has not been genetically modified. Native strains may be isolated from environmental samples. Native strains of most known and industrially relevant bacteria are available from culture collections. In contrast, an engineered bacterial strain is a strain that is genetically modified through any means known in the art. Engineered strains may be modified to show increased production of specific secondary metabolites and/or to show improved growth and/or resistance to antibiotics which may be used for selection.

As used herein, a culture medium is any medium supporting the growth of the respective bacterial culture. As opposed to a minimal medium, a rich medium comprises all nutrients to ensure optimal growth of the bacteria. Suitable media for the growth of different bacteria are well-known in the art.

As used herein, bacterial secondary metabolites are non-essential compounds not needed for growth and proliferation, but that can facilitate the interaction of a microbe with its environment and provide a competitive evolutionary advantage. Bacterial secondary metabolites, including pigments, alkaloids, terpenes, phenolic compounds, and antimicrobial agents, are bioactive compounds that provide microorganisms with a competitive evolutionary advantage. Not being essential for growth and proliferation, such secondary metabolites play, nevertheless, key roles in microbial survival. In the context of this disclosure, preferred bacterial secondary metabolites comprise pigments, alkaloids, terpenes and terpenoids, phenolic compounds, antimicrobial agents such as phenazines, polyketides, and peptide secondary metabolites (e.g., non-ribosomal or ribosomal peptides), and glycosides.

A pigment is a chemical compound displaying a particular colour, particularly an organic compound which typically shows extensive conjugation. Alkaloids are a broad class of naturally occurring organic compounds containing at least one nitrogen atom.

Terpenes are a class of natural compounds with a structure based on isoprene (C₅H₈)-units. Terpenes may be cyclic or acyclic and are classified by the number n of isoprene units, with important classes including monoterpenes (n=2), sesquiterpenes (n=3), diterpenes (n=4), etc.

Terpenoids are terpene-based compounds comprising further functional groups, including but not limited to alcohol, ether, aldehyde, ketone, carboxylic acid and/or carboxylic ester groups. Phenolic compounds comprise at least one aromatic ring directly bonded with one or more hydroxyl groups.

Phenazines are nitrogen-containing heterocyclic compounds with antimicrobial properties. Polyketides are structurally diverse natural products biosynthesised via polyketide synthases and often possess antibiotic, antifungal, or anticancer activities.

Peptide secondary metabolites include both non-ribosomal and ribosomal synthesized peptides with various functions, often acting as antimicrobials. Glycosides are molecules with a sugar bound to another functional group, commonly contributing to signalling, defence, or bioactivity.

An example of secondary metabolites are bacterial pigments, such as violacein, prodigiosin, carotenoids, melanin, pyocyanin, actinorhodin, and zeaxanthin., which contribute to microbial protection against oxidative stress, antimicrobial defence, UV resistance, and quorum sensing. These properties have highlighted their potential for use as natural colourants in textiles and food, antimicrobial, anticancer, and antiviral compounds in pharmaceuticals, and as UV-protective and antioxidant agents in cosmetics.

Production of bacterial pigments and other secondary metabolites is highly dependent on environmental factors such as pH, nutrient availability, oxygen concentration, and carbon source composition. Pigment production generally occurs during the stationary phase of bacterial growth and is often suppressed during exponential growth. Current strategies to increase pigment production include fermentation, genetic engineering, and co-cultivation. During the fermentation process, the temperature, pH, oxygen and nutrient availability of the bacterial culture is optimised to maximise the production of secondary metabolites. Optionally, the production of metabolites may be enhanced with genetic engineering through techniques such as mutagenesis, recombinant DNA technology, or genome editing. Further, multiple bacterial strains may be used in co-cultivation to trigger natural microbial interactions, resulting in an exchange of signalling molecules and metabolites, and thus increasing yields of metabolites. While these methods have demonstrated effectiveness, they are often resource-intensive, costly, and difficult to scale for industrial applications. Genetic modification is a particularly problematic approach as it can take decades to perfect, and faces regulatory constraints that increase production complexity and limit commercial viability. Additionally, environmental manipulation techniques provide only limited control over production of secondary metabolites and frequently result in inconsistent yields.

An example of a model pigment producing bacterium is *Janthinobacterium lividum,* a Gram-negative, motile, aerobic bacterium commonly isolated from various natural environments, such as soil, rivers, lakes, and springs. It is particularly known for producing violacein, a water-insoluble blue-purple pigment. Violacein has been shown to have significant potential, including antibacterial, antiviral, anticancer, antiparasitic, and antioxidant properties, making it a valuable compound for various applications. Current methods to increase violacein yield rely on manipulating environmental conditions or through genetic modifications of the bacterium. The *vio* operon, a set of genes responsible for violacein biosynthesis, is the target for genetic modification in order to increase violacein production. By introducing mutations or overexpressing certain genes, researchers have attempted to increase the flux through the violacein biosynthetic pathway. While these methods offer greater control over pigment synthesis, they are labour-intensive, expensive, and often face regulatory hurdles, particularly when applied in commercial settings where genetically modified organisms are subject to strict regulations. Despite these advances, the cost and complexity associated with genetic modification, combined with the unpredictability of environmental manipulation, make these approaches less than ideal for industrial-scale violacein production. There remains a clear need for a more efficient, scalable, and reliable method for enhancing production of metabolites like violacein.

The present invention addresses this challenge by using plasma-generated RONS to alter bacterial metabolism and increase pigment production. This method provides a rapid, cost-effective, and regulatory-compliant alternative to conventional methods, allowing for enhanced metabolite production without the need for genetic modification or extensive process optimization. Unlike traditional fermentation-based approaches that require prolonged incubation times and strict environmental controls, cold plasma treatment can be applied within seconds to minutes while achieving comparable or superior yields.

Plasma is an ionised gas, referred to as the fourth state of matter, consisting of ions, electrons, and uncharged particles (atoms, molecules, and radicals). Plasma contains an abundance of reactive species: electrons, ions, neutral molecules, atoms, free radicals and charged species, in particular reactive oxygen and nitrogen species. During plasma generation, radiation is emitted in both the UV and near-infrared regions. Different states of plasma can be distinguished by temperature. High temperature plasmas are characterised by the thermal equilibrium of the electrons and ions, in contrast to cold plasma.

Cold plasma, also referred to as non-thermal plasma, is a plasma wherein the temperature of the electrons is higher than the ions and neutrons. In a typical cold plasma, only electrons have high energy while heavy particles like ions, radicals and neutral particles remain close to room temperature. Preferably, the temperature of ions, radicals and neutral particles remains between 20°C and 50°C, more preferably between 20°C and 40°C. Cold plasma is preferred for biological applications, and thus was the type of plasma system used in the present invention.

Cold plasma may be generated through various methods known in the art. Typically, plasma is generated through electrical discharge upon application of high voltages to two separated electrodes. In dielectric barrier discharge (DBD), the electrodes are separated by an insulating dielectric barrier. Plasma may be generated from air or externally supplied gases, at ambient or reduced pressure. As used herein, ambient air refers to air of the atmosphere at any given location where the method of the invention may be performed. The composition of atmospheric air is known to one skilled in the art. Typically, dry air comprises about 78 vol.% N₂, about 21 vol.% O₂, about 0.9 vol.% Ar and about 0.04 vol.% CO₂. Preferably, ambient air has a temperature of around 15-35°C, preferably 15-25°C and a relative humidity of 10-90%, preferably 20-90%, more preferably 30 to 60%.

Plasma treatment of a target of interest may be direct or indirect. In direct plasma treatment, the target to be treated comes into direct contact with the plasma. Direct plasma treatment may be achieved with a volume barrier discharge (VBD) configuration, wherein target serves as second electrode or is placed between the electrodes. Alternatively, direct plasma treatment is also possible with plasma jets, wherein a gas flow transports plasma generated inside a nozzle to the target. In contrast, in indirect treatment, the target to be treated does not come into direct contact with the plasma. In indirect treatment, the target is generally outside the electrode configuration for plasma generation. Plasma may be generated close to the sample, such that reactive species in the plasma, particularly long-lived reactive species, may still interact with the target. A typical device for indirect treatment is a surface barrier discharge (SBD) device, as discussed below. An alternative way to achieve indirect treatment e.g. of biological samples or cells is to expose a medium to plasma, followed by treatment of the biological sample or cells with the medium.

Of particular relevance to the present invention is the SBD, a form of DBD which consists of a dielectric layer separating two metallic electrodes. Plasma is typically generated at the edges of the electrode and spreads across the surface when a sufficiently high potential difference is applied between the two electrodes. It is a simple electrode configuration which has been adopted for biological applications due to its stability in molecular gases, its scalability which ranges from cm² to m², and its ability to generate a large variety of reactive species when operated in ambient air. They are also relatively easy to manufacture, making it a suitable device of choice for a wide range of applications ranging from decontamination in food processing to localised treatment of living cells.

With an indirect SBD cold plasma treatment, the antimicrobial action is based on longer-lived species and recombined species, opposed to the combined action of short-lived species, electrons, free radicals, and positive and negative ions as would be seen with a direct plasma treatment. Despite lack of short-lived species, SBDs have been shown to produce a large amount of RONS when operated in ambient air, including ozone, nitric oxide, nitrous oxide, dinitrogen pentoxide, nitric acid, nitrate, nitrogen dioxide, nitrous acid, hydrogen and hydrogen peroxide. Many of these RONS species have been shown to have antimicrobial activity. For example, ROS induce oxidation of several biomolecules within bacteria including RNA. Oxidation of RNA can result in strand breaks and mutations in the genetic code leading to cell death. However, more recent studies have suggested that subinhibitory exposure to cold plasma can have stimulatory effects on microbial metabolism, potentially causing upregulation of pathways involved in the production of secondary metabolites. This is particularly relevant for bacteria like *Janthinobacterium lividum,* which produce valuable compounds such as violacein. Compared to the genetic modification of bacterial strains, which is time-consuming, costly, and requires regulatory approvals, cold plasma offers a relatively quick, cost-effective, and scalable solution that can be easily integrated into existing microbial fermentation processes.

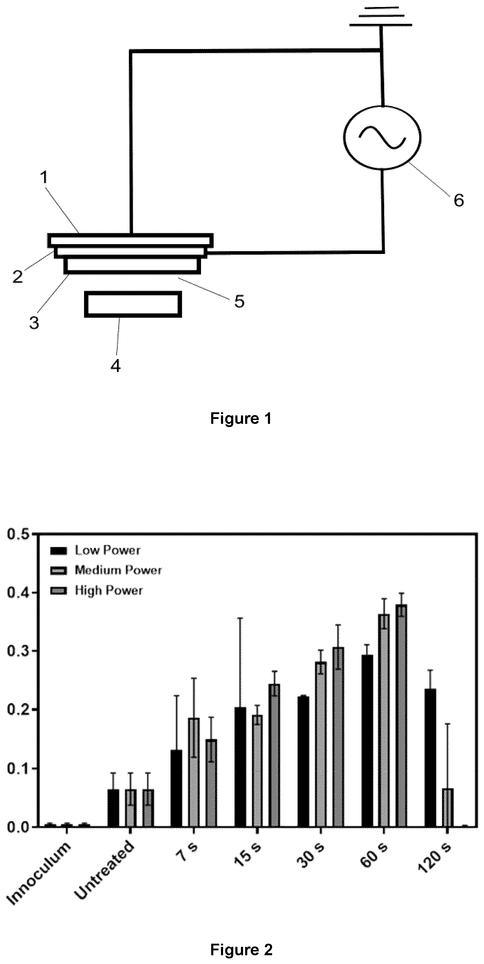

The main objective of the present invention is to provide a method for increasing the production of bacterial secondary metabolites, such as pigments, through the application of plasma-generated RONS. In this invention, an indirect low-temperature SBD cold plasma device is employed for plasma generation. The SBD plasma source comprises a grounded stainless steel mesh electrode (1) featuring hexagonal gaps on the surface of a dielectric material. A quartz plate, measuring 100 x 100 mm and 1 mm in thickness, serves as the dielectric barrier (2) that separates the mesh electrode from a high-voltage copper plate electrode (3). This specific electrode configuration was selected due to its capacity to generate a significant quantity of reactive species capable of antimicrobial activity when operated in ambient air, and its scalability-an important advantage over other electrode configurations. The SBD device described here uses an indirect method, which primarily produces longer-lived reactive species, delivered as the plasma effluent (5) shown in Figure 1. The plasma system is powered by a power supply (6) and function generator, with a custom-designed high-voltage amplifier used to produce a sinusoidal signal with variable voltage. The SBD is placed above the bacterial sample (4).

The electrode configuration described in this invention, while optimized for generating reactive species with antimicrobial and stimulatory effects in ambient air, can be adjusted or modified depending on specific experimental requirements and should not be considered limited to the configuration outlined here. This flexibility allows for tailoring the setup to various applications or scaling needs, enhancing the device's versatility.

The present invention addresses the limitations of current methods of bacterial secondary metabolite production, specifically pigments, by using a novel approach of plasma-generated RONS. This method offers significant advantages, including reduced processing time, lower cost, and scalability, making it practical for industrial applications. The invention describes the increased production of a secondary metabolite, particularly violacein, as a result of exposure to subinhibitory levels of cold plasma while being maintained and grown in specific culture media under defined growth conditions.

Although the invention uses *Janthinobacterium lividum DSM1522,* a Gram-negative, motile, aerobic bacterium commonly isolated from soil and aquatic environments, as an example strain, the method is applicable to other bacterial species capable of producing secondary metabolites, including pigments. The method may be used to produce one or multiple secondary metabolites from a pure culture of one bacterial strain or a coculture of multiple bacterial strains.

Thus, the present invention is described by the following items:

### [Method]

1. A method for producing at least one secondary metabolite, comprising the steps of:
   (i) providing a bacterial culture capable of producing the at least one secondary metabolite,
   (ii) treating the bacterial culture of step (i) with cold plasma, and
   (iii) cultivating the bacterial culture obtained from step (ii).
2. The method of any one of the preceding items, wherein the at least one secondary metabolite is one or more selected from pigments, alkaloids, terpenes, phenolic compounds, resins, toxins and antimicrobial agents.
3. The method of any one of the preceding items, wherein a single secondary metabolite is produced, preferably a single preselected secondary metabolite.
4. The method of any one of the preceding items, wherein the secondary metabolite is a pigment, preferably selected from violacein, prodigiosin, carotenoids, melanin, pyocyanin, actinorhodin, and zeaxanthin, more preferably wherein the secondary metabolite is violacein.
5. The method of any one of the preceding items, wherein the bacterial culture comprises at least one bacterial strain of a genus selected from *Janthinobacterium, Chromobacterium, Pseudomonas, Serratia, Streptomyces,* Micrococcus or Paracoccus spp.
6. The method of any one of the preceding items, wherein the bacterial culture comprises at least one bacterial strain selected from *Janthinobacterium lividum, Chromobacterium violaceum, Pseudomonas aeruginosa, Serratia marcescens, Streptomyces coelicolor, Micrococcus luteus, Paracoccus zeaxanthinifaciens.*
7. The method of any one of the preceding items, wherein the bacterial culture comprises *Janthinobacterium lividum,* preferably wherein the bacterial culture comprises *Janthinobacterium lividum* DSM1522.
8. The method of any one of the preceding items, wherein the secondary metabolite is violacein and the bacterial culture is a culture of *Janthinobacterium lividum,* preferably *Janthinobacterium lividum* DSM1522.
9. The method of any one of the preceding items, wherein the bacterial culture is a co-culture or a pure culture, preferably a pure culture comprising a single bacterial strain.
10. The method of any one of items 5-9, wherein the at least one bacterial strain is a native or engineered bacterial strain, preferably a native bacterial strain.
11. The method of any one of the preceding items, wherein the bacterial culture is a liquid bacterial culture.
12. The method of any one of the preceding items, wherein the bacterial culture is provided in a culture medium, wherein the culture medium is preferably a rich culture medium.
13. The method of item 12, wherein the culture medium is a medium selected from a tryptic soy broth (TSB) medium, brain-heart infusion broth, Luria-Bertani or lysogeny broth, and nutrient broth, preferably wherein the culture medium is a tryptic soy broth medium.
14. The method of item 12 or 13, wherein the medium further comprises at least one substrate for production of the secondary metabolite, such as glycerol, in particular 1% glycerol, amino acids, preferably L-tryptophan and/or antibiotics, preferably ampicillin. For the production of violacein, the addition of glycerol, L-tryptophan and/or ampicillin, in particular 1% glycerol is in particular preferred.
15. The method of any one of the preceding items, wherein the bacterial culture is grown for 12 to 30 hours, preferably about 24 hours before performing step (ii).
16. The method of any one of the preceding items, wherein the bacterial culture provided in step (i) is in stationary phase of growth.
17. The method of any one of the preceding items, wherein cold plasma treatment in step (ii) is indirect.
18. The method of item xx, wherein step (ii) comprises exposing the surface of the bacterial culture to cold plasma, preferably wherein the cold plasma is generated at a distance of 2-8 mm, preferably 4-6 mm, most preferably 5 mm to the surface of the bacterial culture.
19. The method of any one of the preceding items, wherein the cold plasma comprises reactive oxygen and nitrogen species (RONS).
20. The method of item 19, wherein the RONS comprise one or more selected from the group consisting of ozone, hydroxyl radicals, nitric oxide, nitrous oxide, dinitrogen pentoxide, nitric acid, nitrite, nitrate, nitrogen dioxide, nitrous acid, and/or hydrogen peroxide.
21. The method of any one of the preceding items, wherein the cold plasma is generated in ambient air, preferably with a surface barrier discharge (SBD) device.
22. The method of any one of the preceding items, wherein the cold plasma is low-power, medium-power or high-power plasma, preferably medium-power or high-power plasma, more preferably high-power plasma, wherein low-power plasma corresponds to 20 V and 16.7 W, medium-power plasma corresponds to 26 V and 30 W, and high-power plasma corresponds to 33 V and 43 W, with a frequency of 48 kHz.
23. The method of any one of the preceding items, wherein cold plasma treatment in step (ii) is performed with an exposure time of 7 to 120 seconds, preferably 15 to 90 seconds, more preferably 30 to 60 seconds, wherein the treatment is performed once, with the culture stirred at room temperature during the treatment.
24. The method of any one of the preceding items, wherein cell viability is maintained in step (ii).
25. The method of any one of the preceding items, wherein cold plasma treatment in step (ii) is subinhibitory or inhibitory, preferably subinhibitory.
26. The method of any one of the preceding items, wherein the growth rate of the bacterial culture in step (iii) is reduced compared to an untreated bacterial culture.
27. The method of any one of the preceding items, wherein the bacterial culture is cultivated for 12 to 96 hours, preferably for 24 to 72 hours in step (iii).
28. The method of any one of the preceding items, wherein the bacterial culture is cultivated under optimal growth conditions in step (iii) and/or wherein the bacterial culture provided in step (i) is cultivated under optimal growth conditions from its inoculation until step (ii).
29. The method of item 28, wherein optimal growth conditions comprise one or more of:
   a. a temperature of 20°C to 30°C, more preferably 23°C to 27°C, particularly 25°C,
   b. agitation of the culture, preferably through shaking at 150 to 250 rpm, and
   c. a pH of 6 to 8, preferably 6.5 to 7.5,
   particularly wherein optimal growth conditions comprise all of a-c.
30. The method of any one of the preceding items, wherein the bacterial culture is kept in the same medium in steps (i)-(iii).
31. The method of any one of the preceding items, further comprising after step (iii) a step of
   (iv) extracting the at least one secondary metabolite from the bacterial culture obtained in step (iii).
32. The method of item 31, wherein extracting the secondary metabolite comprises centrifugation of the bacterial culture, and wherein the secondary metabolite is extracted from the cell pellet and/or from the supernatant.
33. The method of item 32, wherein centrifugation is performed with one or more of the following parameters:
   a. a speed of 9660-10000 x g,
   b. a temperature of 4-7°C, and
   c. a centrifugation time of 10-15 min,
   preferably wherein centrifugation is performed with all parameters a-c.
34. The method of any one of items 31-33, wherein the secondary metabolite is extracted with an organic solvent, wherein the organic solvent is preferably an alcohol, such as butanol, methanol or ethanol, preferably butanol.
35. The method of any one of items 31-34, wherein extracting the secondary metabolite from the bacterial culture comprises the steps of:
   (iv)-a1 mixing the bacterial culture obtained in step (iii) with an organic solvent, preferably an organic solvent as defined in item 34, to obtain a mixture, and (iv)-a2 centrifugation of the mixture, preferably using centrifugation parameters as defined in item 33, and
   (iv)-a3 obtaining the secondary metabolite from the supernatant.
36. The method of any one of items 31-34, wherein extracting the secondary metabolite from the bacterial culture comprises the steps of:
   (iv)-b1 centrifugation of the bacterial culture obtained in step (iii), preferably using centrifugation parameters as defined in item 33, and
   (iv)-b2 lysing the cell pellet with a lysing agent, preferably with SDS, particularly 10% SDS, and
   (iv)-b3 adding an organic solvent to the lysate, preferably an organic solvent as defined in item 34, and
   (iv)-b4 obtaining the secondary metabolite from the solution.
37. The method of any one of items 31-36, further comprising purifying the at least one secondary metabolite obtained in step (iv).

### [Use]

38. Use of cold plasma to increase production of at least one secondary metabolite in a bacterial culture.
39. Use according to item 38, wherein the at least one secondary metabolite is a secondary metabolite as defined in any one of items 2-4.
40. Use according to item 38 or 39, wherein the bacterial culture is a bacterial culture as defined in any one of items 5 to 16.
41. Use according to any one of items 38 to 40, wherein the cold plasma is a cold plasma as defined in any one of items 19 to 22.

### [System]

42. A system for increasing production of at least one secondary metabolite in a bacterial culture, comprising
   (A) a surface barrier discharge (SBD) cold plasma source, and
   (B) a vessel containing a liquid bacterial cell culture,
   wherein the vessel (B) is arranged below the SBD cold plasma source (A), and wherein the surface of the liquid bacterial cell culture is exposed to the plasma-generating surface of the SBD cold plasma source (A).
43. The system of item 42, wherein the distance between the surface of the liquid bacterial cell culture and the plasma-generating surface of the SBD cold plasma source (A) is of 2-8 mm, preferably 4-6 mm, most preferably 5 mm.
44. The system of item 42 or 43, wherein the SBD cold plasma source is configured to generate plasma from ambient air.
45. The system of any one of items 42-44, wherein the SBD cold plasma source (A) comprises a grounded mesh electrode (1), a high-voltage plate electrode (3) and a dielectric barrier (2) arranged between the grounded mesh electrode (1) and the high-voltage plate electrode (3), and a power supply (6) connected to the grounded mesh electrode (1) and the high-voltage plate electrode (3).
46. The system of any one of items 42-45, wherein the grounded mesh electrode (1) is made of stainless steel, copper, aluminium, or any other conductive material, preferably stainless steel.
47. The system of any one of items 42-46, wherein the grounded mesh electrode (1) forms gaps on the surface of the dielectric barrier (2), wherein the gaps preferably have the shape of a regular hexagonal mesh, particularly with a distance in the in the range of 3 to 9 mm, preferably 5 to 7 mm, particularly 6 mm.
48. The system of any one of items 42-47, wherein the high-voltage plate electrode (3) is made of copper, aluminum, stainless steel or any other conductive material, preferably copper.
49. The system of any one of items 42-48, wherein the dielectric barrier (2) is made of quartz, plastic, ceramic, or any other suitable material that is not conductive and has thermal resistance, preferably quartz.
50. The system of any one of items 42-49, wherein the dielectric barrier has a thickness of 0 to 5 mm, preferably 1 mm.
51. The system of any one of items 42-50, wherein the power supply (6) comprises a function generator and a high-voltage amplifier.
52. The system of any one of items 42-51, wherein the bacterial culture is a bacterial culture as defined in any one of items 5-16.

Depending on the bacterial strain(s), suitable growth conditions may vary. Standard growth conditions known in the art are generally preferred for each respective bacterial strain. As used herein, optimal growth conditions are conditions which support growth of the bacteria without any particularly limiting factor. Optimal growth conditions comprise parameters such as temperature, media composition including nutrients, pH, agitation and oxygenation. Extraction and purification methods for the at least one secondary metabolite depend on the metabolite and may be chosen from known extraction and purification methods by one skilled in the art. The extraction protocol may vary as described in the items depending on whether the secondary metabolite is predominantly present in the cells or excreted. Suitable downstream purification methods include but are not limited to filtration, Soxhlet extraction, crystallization and high performance liquid chromatography and are known to one skilled in the art.

In preferred embodiments of the present invention, the bacterium, particularly *Janthinobacterium lividum* DSM1522, is maintained and grown in specific culture media under defined growth conditions. The inoculation of *Janthinobacterium lividum* DSM1522 is in TSB medium at around pH 7 and at 25°C for 24 hrs. The growth medium consists of 2.5g glucose monohydrate, 5g sodium chloride, 2.5g dipotassium hydrogen phosphate, 17g pancreatic digest of casein, 3g papaic digest of soyabean meal per 1 L of distilled water. The pH of the culture medium ranges is 7.3 ± 0.2, with the optimal range being between 6.5 and 7.5. The bacterial culture is treated with cold plasma-generated RONS. The ideal temperature for bacterial growth is maintained at 25°C, and the culture is incubated for 24 to 72 hours under shaking conditions (150 to 250 rpm). Once confluence is reached, the bacterial biomass is separated from the liquid culture medium by centrifugation at the speed in the range of 9,660 x g to 10,000 x g at a temperature ranging from 4 to 7 °C for a period in the range of 10 to 15 minutes. The cell pellet was lysed with 10 % SDS. After 5 min, water saturated butanol (1:2 v/v) was added. The concentration was estimated with measuring absorbance at 585 nm.

As part of the invention, *Janthinobacterium lividum* DSM1522 was exposed to a range of cold plasma treatments, both inhibitory and subinhibitory. As used herein, inhibitory treatment refers to a treatment causing inhibition of bacterial growth for at least 24 h. Subinhibitory treatment does not cause full inhibition of bacterial growth, but may reduce the rate of bacterial growth. The experimental results show that exposing *Janthinobacterium lividum* DSM1522 to subinhibitory cold plasma treatments significantly increases violacein production. Specifically, the results indicate that subinhibitory plasma treatments resulted in an increased yield of violacein compared to untreated controls, with the magnitude of increase depending on the power of the plasma and the duration of exposure. The yield of the violacein pigment produced is 5.83 times higher than baseline when *Janthinobacterium lividum* DSM1522 maintained in TSB is exposed to high power plasma for 60 s (Figure 2).

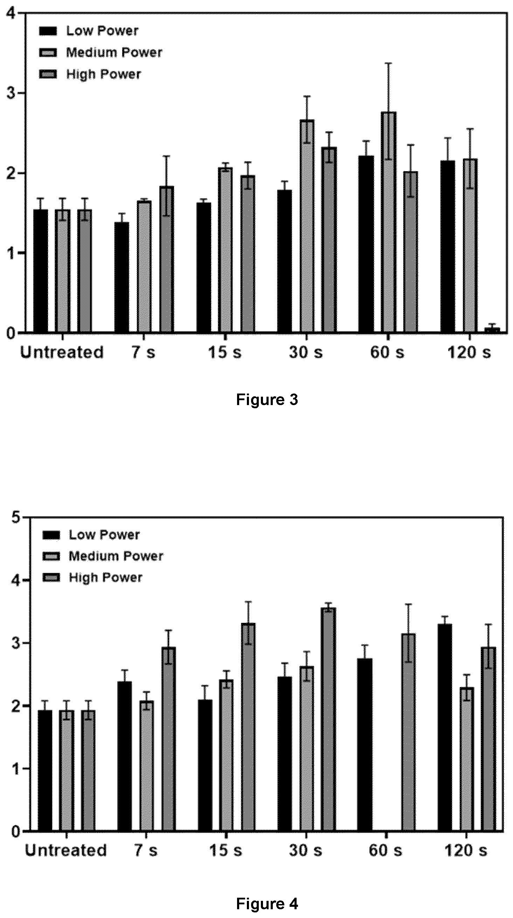

Further, *Janthinobacterium lividum* DSM1522 has been exposed to low, medium and high-power plasma and is maintained in a growth medium at pH 7.3 ± 0.2 at 25°C for a period of 48 or 72 hours. Increased pigment production is still observed after 48 (Figure 3) and 72 hrs (Figure 4).

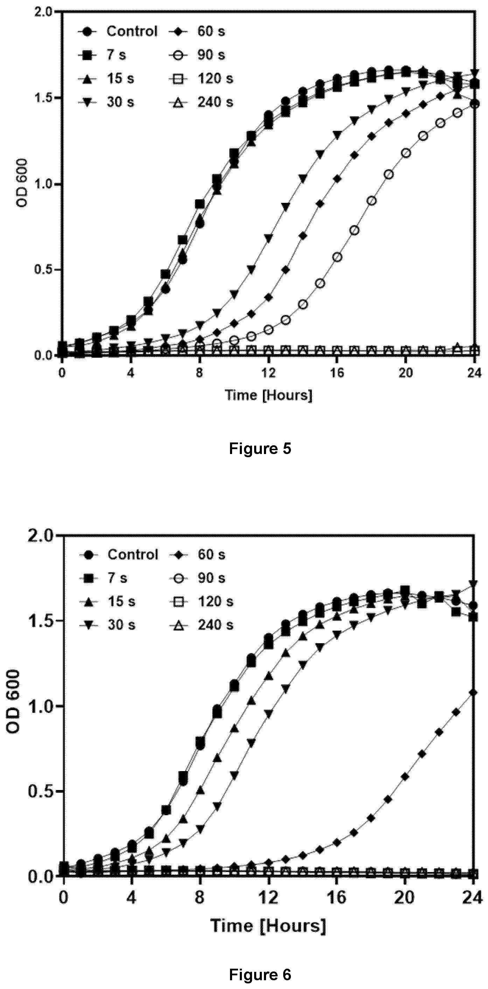

Further, growth of *Janthinobacterium lividum* DSM1522 in response to low (Figure 5) and high-power (Figure 6) cold plasma exposure is monitored over 24 hrs. High power plasma has a greater effect of growth of *Janthinobacterium lividum* DSM1522 than low power. Results demonstrated that the majority of plasma treatments did slow the bacterial growth rate but did not have a completely inhibitory effect, confirming the subinhibitory nature of the treatments.

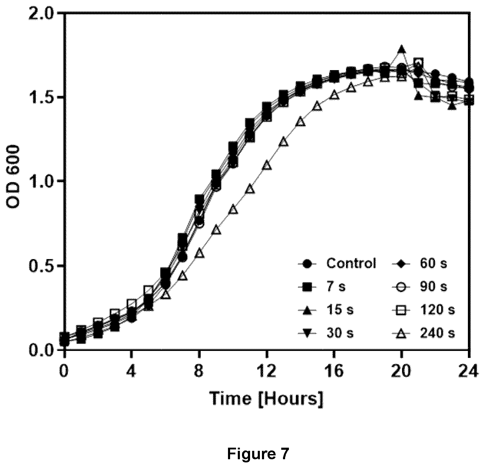

As a comparison, the effect of UV exposure on growth of *Janthinobacterium lividum* DSM1522 was measured over a 24-hour time period (Figure 7). UV is explored as a method for increasing violacein production as it is known to induce a bacterial stress response which could lead to a decreased growth rate and an increased production of secondary metabolites. However, the results of the present invention indicate that cold plasma is more effective than UV at disrupting bacterial metabolism, which suggests cold plasma would be more effective at increasing secondary metabolite production than UV. While both methods trigger stress responses within bacteria, cold plasma produces a broader range of reactive species, which may explain its superior performance in enhancing pigment production. UV alone does not affect growth in comparison with cold plasma.

The primary advantage of the present invention is the increased production of bacterial secondary metabolites through exposure to plasma-generated RONS. This was shown through the example of violacein production in *Janthinobacterium lividum,* wherein violacein production was increased by a factor of up to 5.83. This method offers a rapid, scalable, and cost-effective alternative to traditional approaches, making it suitable for industrial applications. The process is simple, requiring no genetic modifications and minimal changes to standard bacterial culture protocols. The method can be applied to other bacterial strains, other secondary metabolites and with alternative culture media, as defined in the items above. It has potential for use in various industrial and biomedical fields.

Thus, the advantages provided by the present invention include but are not limited to:
1. Higher production of secondary metabolites in bacteria in response to exposure to plasma-generated RONS, such as of violacein in *Janthinobacterium lividum* DSM1522.
2. Effective at short exposure times (seconds), minimising the overall processing time and energy costs of pigment production and increasing efficiency.
3. After exposure to plasma-generated RONS, the pigment production is sustained over at least a 72-hour period.
4. Simple growth requirements for growing and maintaining the bacterium both before after RONS exposure. In particular, optimal growth conditions known for the respective bacterial strains may be used, and there is no need for time-consuming optimization or complex and costly control of growth conditions to enhance secondary metabolite production.
5. The present invention does not require complex genetic modification, but may optionally be used to increase secondary metabolite production also in engineered strains.
6. Exogenous precursors, such as L-Tryptophan, are not required for the increased production of secondary metabolites, such as violacein. However, precursors may be added to increase secondary metabolite production in a synergistic manner with the method of the present invention.
7. The pigment extraction process after plasma treatment is simple.
8. Increased pigment production when exposed to plasma-generated RONS compared to other stimuli, such as UV exposure.

The present method can be implemented by a skilled professional using a system or device according to the invention, which is specifically designed to apply cold plasma to bacterial cultures. Preferred embodiments of the method may involve configuring various parts of the apparatus to achieve the optimal plasma conditions and other parameters described herein.

The features disclosed in the preceding description including the items, in the following claims, or accompanying drawings - whether expressed in specific terms or as means for performing the described functions, or methods for achieving the disclosed results - may be used independently or in combination to carry out the invention in various forms.

While the invention has been described with reference to specific example embodiments, many equivalent modifications and variations will be evident to those skilled in the art upon review of this disclosure. Therefore, the embodiments provided are illustrative and not limiting. Various modifications may be made without departing from the spirit and scope of the invention.

For clarity, all theoretical explanations are included to enhance the reader's understanding. Section headings are used for organizational purposes only and should not limit the content described.

### EXAMPLES

The following examples are given by way of illustration and therefore should not be construed to limit the scope of the present invention.

### EXAMPLE 1

### EFFECT OF LOW, MEDIUM AND HIGH-POWER COLD PLASMA TREATMENT ON VIOLACEIN PRODUCTION

*Janthinobacterium lividum* DSM1522 was grown for 24 hours on TSA and a loopful was taken to inoculate 50 mL of growth medium in a 250 mL Erlenmeyer flask, with the pH being around 7. The flasks were incubated at 25°C in a rotary shaker at 200 rpm for 24 hours. Violacein pigment was observed within 6 hours of incubation and continued to be produced during biomass increase. The bacterial cultures were treated using the cold plasma device depicted in Figure 1 at low (20 V, 16.7 W), medium (26 V, 30 W), and high (33 V, 43 W) power levels, all at a frequency of 48 kHz. These levels included both subinhibitory and inhibitory treatments. Treatments were conducted for 7, 15, 30, 60, and 120 seconds. After 24 hours of cultivation, the bacterial culture was harvested by centrifugation at 10,000 × g for 10 minutes at 4°C. The supernatant was discarded, and the resulting cell pellet, containing both biomass and violacein pigment, was retained. Biomass concentration was estimated by measuring the optical density (OD) at 600 nm. The pellet was resuspended in 10% (w/v) SDS and incubated at room temperature for 5 minutes to lyse the cells. Following lysis, water-saturated butanol was added at a 1:2 (v/v) ratio to extract violacein. The mixture was vortexed briefly and centrifuged again at 10,000 × g for 10 minutes. The supernatant containing violacein was carefully collected. Violacein concentration in the butanol extract was quantified by measuring absorbance at 585 nm, using an extinction coefficient (ε) of 0.05601 mL·µg⁻¹·cm⁻¹. The yield was calculated as approximately 1.0 g of violacein per gram of dry biomass after 24 hours of cultivation. The pigment showed maximum absorption at 585 nm in the UV-VIS spectra, characteristic of violacein. All plasma conditions resulted in an increase in violacein production. A subinhibitory high-power plasma treatment resulted in 5.83 times increase in violacein production. Long treatment times at high power resulted in an inhibitory effect and caused and decrease in pigment production.

### EXAMPLE 2

### EFFECT OF INCUBATION TIME ON VIOLACEIN PRODUCTION

The impact of different incubation times (24, 48, and 72 hours) on violacein production was studied. Cultures of *Janthinobacterium lividum* DSM1522 were treated in the same manner as in Example 1 with low, medium and high-power plasma. Cultures were then left to incubate for 48 and 72 hours. Increased violacein production was sustained for cultures treated with high power plasma over a 72-hour incubation period.

### EXAMPLE 3

### EFFECT OF COLD PLASMA ON GROWTH

The effect of low- and high-power cold plasma treatments on growth of *Janthinobacterium lividum DSM1522* was evaluated over a 24-hour period. The results demonstrated that low power plasma treatments did slow growth of the bacterium, however high-power plasma treatments were more disruptive to the growth of the bacterium.

### EXAMPLE 4

### COMPARISON OF COLD PLASMA AND UV TREATMENTS

UV was tested as an alternative method for increasing violacein production. UV light induces oxidative stress and DNA damage, which can potentially trigger the production of secondary metabolites. However, cold plasma was found to be more effective than UV in increasing violacein yield. Cold plasma generates a broader range of reactive species, including ROS and RNS, which likely contribute to its superior performance in stimulating pigment production.

## Claims

1. A method for producing at least one secondary metabolite, comprising the steps of:
(i) providing a bacterial culture capable of producing the at least one secondary metabolite,
(ii) treating the bacterial culture of step (i) with cold plasma, and
(iii) cultivating the bacterial culture obtained from step (ii).

2. The method of claim 1,
wherein the at least one secondary metabolite is preferably one or more selected from pigments, alkaloids, terpenes, phenolic compounds, resins, toxins and antimicrobial agents,
wherein the secondary metabolite is more preferably a pigment,
in particular a pigment selected from violacein, prodigiosin, carotenoids, melanin, pyocyanin, actinorhodin, and zeaxanthin.

3. The method of any one of the preceding claims,
wherein the bacterial culture is a co-culture or a pure culture, preferably a pure culture comprising a single bacterial strain,
wherein the bacterial culture preferably comprises at least one bacterial strain of a genus selected from *Janthinobacterium, Chromobacterium, Pseudomonas, Serratia, Streptomyces,* Micrococcus or Paracoccus spp.,
wherein the bacterial culture more preferably comprises at least one bacterial strain selected from *Janthinobacterium lividum, Chromobacterium violaceum, Pseudomonas aeruginosa, Serratia marcescens, Streptomyces coelicolor, Micrococcus luteus, Paracoccus zeaxanthinifaciens,* in particular *Janthinobacterium lividum* DSM1522.

4. The method of any one of the preceding claims,
wherein the bacterial culture is a liquid bacterial culture, and/or wherein the culture medium is preferably a medium selected from a tryptic soy broth (TSB) medium, brain-heart infusion broth, Luria-Bertani or lysogeny broth, and nutrient broth, particularly wherein the culture medium is a tryptic soy broth medium, wherein the medium optionally further comprises a substrate for production of the secondary metabolite, such as glycerol.

5. The method of any one of the preceding claims,
wherein the cold plasma is generated in ambient air, preferably with a surface barrier discharge (SBD) cold plasma source,
wherein the cold plasma is preferably low-power, medium-power or high-power plasma, more preferably high-power plasma, wherein high-power plasma preferably corresponds to 33 V and 43 W, with a frequency of 48 kHz,
and/or
wherein the cold plasma preferably comprises reactive oxygen and nitrogen species (RONS),
wherein the RONS preferably comprises one or more selected from the group consisting of ozone, hydroxyl radicals, nitric oxide, nitrous oxide, dinitrogen pentoxide, nitric acid, nitrite, nitrate, nitrogen dioxide, nitrous acid, and/or hydrogen peroxide.

6. The method of any one of the preceding claims,
wherein cold plasma treatment in step (ii) is indirect, and/or
wherein step (ii) comprises exposing the surface of the bacterial culture to cold plasma, wherein the cold plasma is preferably generated at a distance of 5 mm to the surface of the bacterial culture, and/or
wherein cold plasma treatment in step (ii) is preferably performed with an exposure time of 7 to 120 seconds, preferably 15 to 90 seconds, more preferably 30 to 60 seconds.

7. The method of any one of the preceding claims, wherein cold plasma treatment in step (ii) is subinhibitory or inhibitory, preferably subinhibitory.

8. The method of any one of the preceding claims,
wherein the bacterial culture is grown for 12 to 24 hours, preferably 24 hours before performing step (ii), and/or
wherein the bacterial culture provided in step (i) is in stationary phase of growth, and/or wherein the bacterial culture is cultivated for 12 to 96 hours, preferably for 24 to 72 hours in step (iii), and/or
wherein the bacterial culture is cultivated under optimal growth conditions in step (iii) and/or wherein the bacterial culture provided in step (i) is cultivated under optimal growth conditions from its inoculation until step (ii),
wherein optimal growth conditions preferably comprise one or more of:
a. a temperature of 20°C to 30°C, more preferably 23°C to 27°C, particularly 25°C,
b. agitation of the culture, preferably through shaking at 150 to 250 rpm, and
c. a pH of 6 to 8, preferably 6.5 to 7.5,
particularly wherein optimal growth conditions comprise all of a-c.

9. The method of any one of the preceding claims,
further comprising after step (iii) a step of
(iv) extracting the at least one secondary metabolite from the bacterial culture obtained in step (iii), and optionally
(v) purifying the at least one secondary metabolite obtained in step (iv),
wherein extracting the secondary metabolite preferably comprises centrifugation of the bacterial culture, and wherein the secondary metabolite is preferably extracted from the cell pellet and/or from the supernatant, and/or
wherein the secondary metabolite is preferably extracted with an organic solvent, wherein the organic solvent is preferably an alcohol, such as butanol, methanol or ethanol, preferably butanol.

10. Use of cold plasma to increase production of at least one secondary metabolite in a bacterial culture.

11. Use according to claim 10,
wherein the at least one secondary metabolite is a secondary metabolite as defined in claim 2, and/or
wherein the bacterial culture is a bacterial culture as defined in any one of claims 3-4, and/or
wherein the cold plasma is a cold plasma as defined in claim 5.

12. A system for increasing production of at least one secondary metabolite in a bacterial culture, comprising
(C) a surface barrier discharge (SBD) cold plasma source, and
(D) a vessel containing a liquid bacterial cell culture,
wherein the vessel (B) is arranged below the SBD cold plasma source (A), and wherein the surface of the liquid bacterial cell culture is exposed to the plasma-generating surface of the SBD cold plasma source (A),
wherein the distance between the surface of the liquid bacterial cell culture and the plasma-generating surface of the SBD cold plasma source (A) is preferably 5 mm.

13. The system of claim 12, wherein the bacterial culture is a bacterial culture as defined in any one of claims 3-4.

14. The system of claim 12 or 13, wherein the SBD cold plasma source is configured to generate plasma from ambient air.

15. The system of any one of claims 12-14, wherein the SBD cold plasma source (A) comprises a grounded mesh electrode (1), a high-voltage plate electrode (3) and a dielectric barrier (2) arranged between the grounded mesh electrode (1) and the high-voltage plate electrode (3), and a power supply (6) connected to the grounded mesh electrode (1) and the high-voltage plate electrode (3),
wherein the grounded mesh electrode (1) preferably forms gaps on the surface of the dielectric barrier (2),
wherein the gaps preferably have the shape of a regular hexagonal mesh, particularly with a distance in the in the range of 3 to 9 mm, and/or
wherein the dielectric barrier has a thickness of 0 to 5 mm, preferably 1 mm.
